# EUROPEAN PATENT APPLICATION

(11) **EP 0 988 844 A2**
(43) Date of publication of application: **29.03.2000**
(21) Application number: 99106199.5
(22) Date of filing: 09.04.1999
(51) Int. Cl.: A61F 9/007

(54) **Apparatus for intubation of lacrimal duct**

(30) Priority: 27.08.1998 JP 25610998
(71) Applicant: MLC Limited Company, Hamamatsu-shi, Shizuoka-ken (JP)
(72) Inventor: Kurihashi, Katsuaki, Hamamatsu-shi, Shizuoka-ken (JP)
(74) Representative: Steil, Christian, Dipl.-Ing.

(57) **Abstract**

An apparatus for intubation of a lacrimal duct (1-12) is inserted into the lacrimal duct (1-12), having a prescribed length of a thinner tube or rod (40), and a prescribed length of a thicker tube (42) which is connected with one end of the thinner tube or rod (40), and a stopper (P; 23; 22, 23) which consists of a plug (P), a brim (23) or a ring (23, 24) and so on, attached to the other end of the thinner tube or rod (40). The tip of the thicker tube (42) is a closed end (53).

## Description

### TECHNICAL BACKGROUND

This invention relates to an apparatus for intubation of the lacrimal duct (lacrimal drainage pathway) for treatments of lacrimal duct obstruction and dry eye.

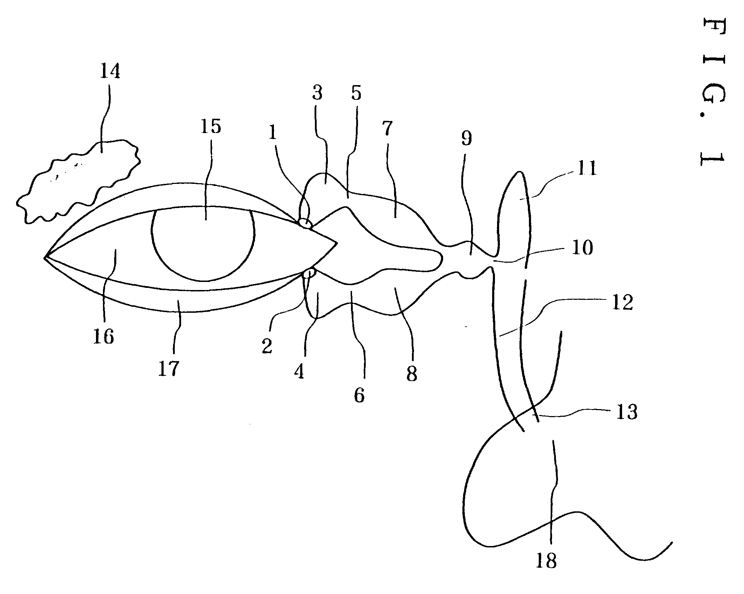

As shown in FIG.1, the lacrimal gland **14** secrete tears which drain into the inferior nasal meatus **18** via the lacrimal duct after moistening the ocular surface **17** having the cornea **15** and conjunctiva **16**. The lacrimal duct consists of the upper punctum **1**, lower punctum **2**, vertical portion of the upper puctum **3**, vertical portion of the lower punctum **4**, boundary portion between the upper vertical and horizontal portions **5**, boundary portion between the lower vertical and horizontal portions **6**, upper horizontal portion **7**, lower horizontal portion **8**, common canaliculus **9**, internal common punctum **10**, lacrimal sac **11**, nasolacrimal duct **12**. The lower end **13** of the nasolacrimal duct **12** opens into the inferior nasal meatus **18**.

In patients with dry eye having hypofunction of the lacrimal gland and deficiency of tears, tears which are very important for eye immediately drain away via the lacrimal duct.

To suppress the tear drainage, occlusion of the upper punctum **1** and/or lower punctum **2** using electric cautery is performed. Occlusion using punctal plug (mentioned later) which inserted into the upper punctum **1** and lower punctum **2** is also performed.

By blocking the upper punctum **1** and lower punctum **2** like this, tears are accumulated in the conjunctival sac and dry eye symptoms disappear in many cases.

Dry eye symptoms include asthenopia, waking irritation, grittiness, foreign body sensation, scratchiness, soreness, difficulty to open the eyes in an air conditioning room, injection, burning and so on.

Recently, aggravation of dry eye symptoms by spending time in front of a monitor is under consideration. This is due to the fact that evaporation of tears is accelerated in individuals with tear deficiency by decreased frequency of blinking which is induced by looking at monitor.

Dropping of artificial tears is performed for the treatment of dry eye. But the ingredients of artificial tears are far from those of natural tears. It is best for eye to be wet with natural tears. Therefore, the treatment of punctal occlusion is superior.

Unlike artificial tears, tears contain lysozyme, lactoferrin, immunoglobulin, and so on which protect eye from bacterias and viruses. And some of artificial tears contain preservative which is harmful to eye.

As other roles of tears, there are an optical role wherein tears make smooth the microscopically irregular surface of the cornea **15** improve eyesight, a role of lubricant wherein tears act as lubricant and the movements of eyelids become smooth, and other roles. Artificial tears can not be expected to play these various roles.

Therefore, occlusion of the upper punctum punctum **1** and/or lower punctum **2** to wet eye with natural tears is superior. But punctal occlusion by argon laser may induce epiphora postoperatively. In such a case, punctal and canalicular surgery are needed to reconstruct canaliculi and puncta.

The method using punctal plug is superior because punctal plug can be removed easily in such cases.

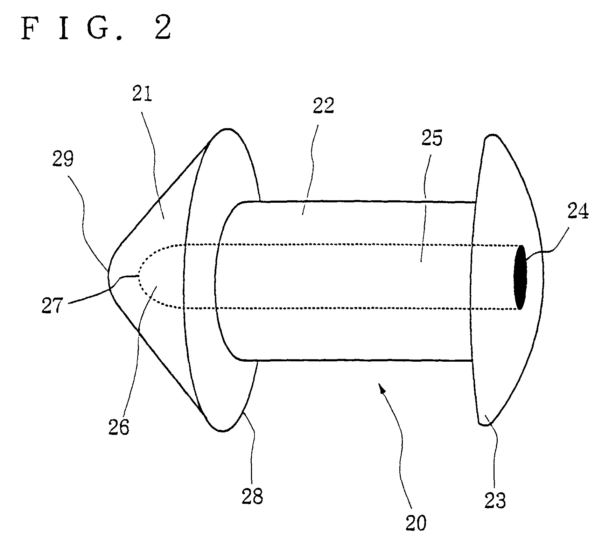

From this point of view, in 1975 Freeman reported a punctal plug as shown in FIG 2 for the treatment of dry eye. For example, see Freeman, JM : The punctum plug : evaluation of a new treatment for the dry eye. Trans Am Acad Ophthalmol Otolaryngol 79 : op 874-879, 1975.

The punctal plug shown in FIG 2 consists of the tip **21**, shaft **22**, brim **23** and there is a hol**e 24** in the center of brim **23**. The hole **24** is continuous with a tubular lumen **25** of shaft **22** and the lumen **26** with a closed end **27** of the tip **21**. The puntal plug shown in FIG.2 measures 2.8mm in total length, in which 1.5∼2.0mm in diameter of brim, 0.7mm in height of brim, 1.5mm in length of shaft and 0.7mm in diameter of shaft.

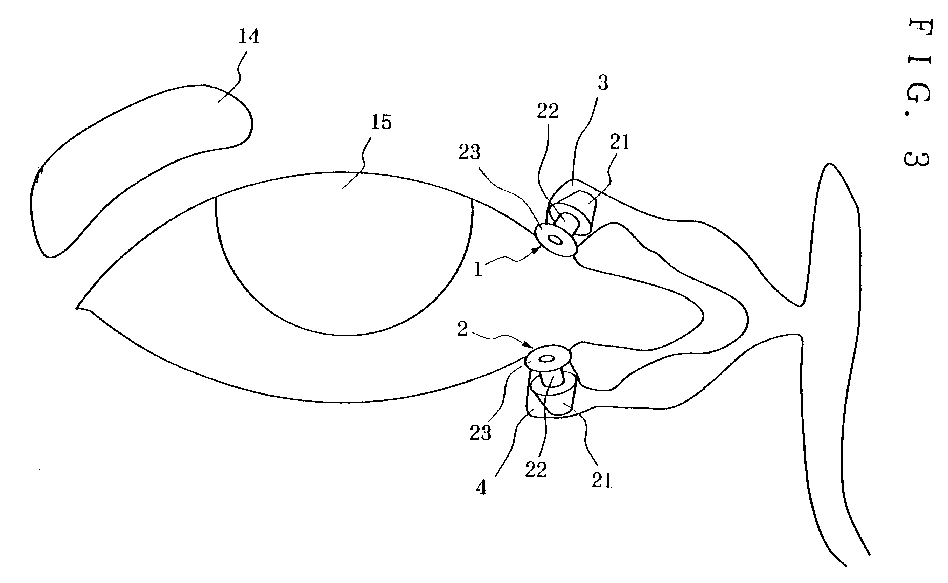

The punctal plug in FIG 2 is used as shown in FIG 3. Punctal plug is inserted into puncta **1, 2** and vertical portion of canaliculus **3**, **4**, and the total length of the puncta **1, 2** and vertical portions of canaliculus **3, 4** is 2.5mm on the average. Therefore, the total length 2.8mm of the punctal plug is too long. Consequently, the brim **23** touches the cornea **28** and not infrequently induces foreign body sensation.

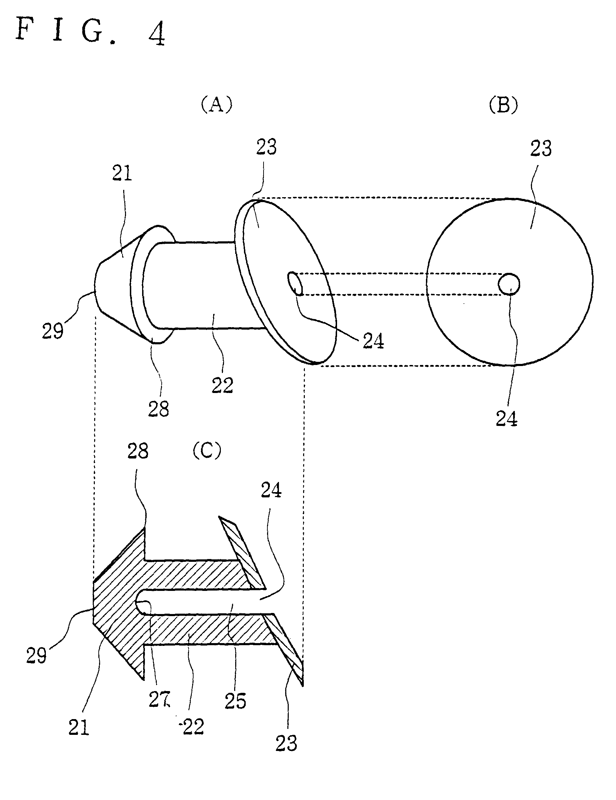

FIG 4 shows a punctal plug of the FCI company. This is used for the treatment of dry eye in Japan also. For example see, Junzo Hirano & Miki Hirano : Experience of the treatment for a case with Stevens-Johnson syndrome with severe keratoconus, Japanese Review of Clinical Ophthalmology 91:41-44, 1997.

The punctal plug in FIG 4 is a miniaturized one. This punctal plug measures 1.7mm in total length, 1.5mm in diameter of brim **23**, and is miniaturized as a whole. It measures 0.1mm in thickness of brim **23** which inclines 20° against the shaft **22**.

The Punctal plug in FIG 4 also consists of tip **21**, shaft **22** and brim **23**, and as in the punctal plug as shown in FIG 2, hole **24** is continuous with the lumen **25** with closed end **27** of shaft **25**.

In case of usage, the tip **29** of punctal plug is pushed into the lacrimal duct until the boundary portion **5, 6** between the vertical portion **3, 4** and horizontal portion **7, 8** of canaliculus or near the boundary portion **5, 6** by a metal probe which is inserted from the hole **24** till the closed end **27**.

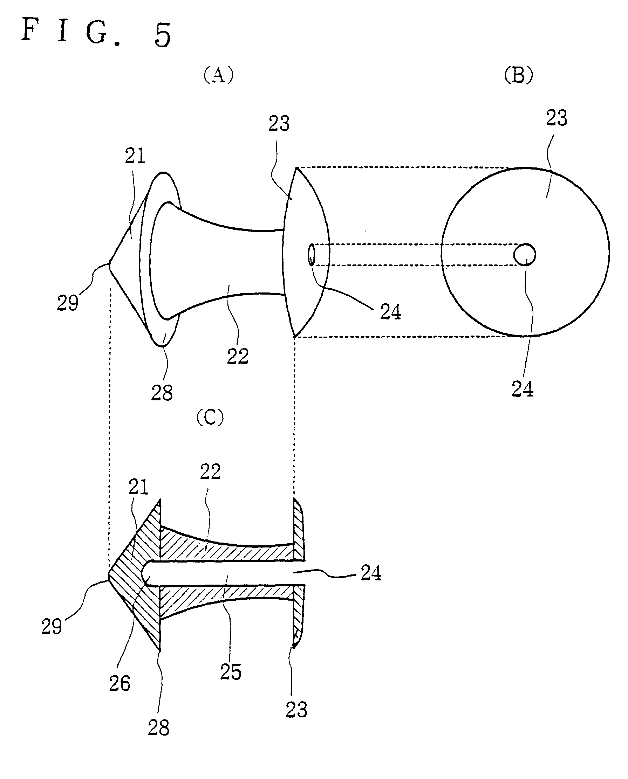

FIG 5 shows a punctal plug with a tapered shaft form. This plug is also miniaturized and consists of the tip **21**, shaft **22** and brain **23.** As in the punctal plug shown in FIG 2, the hole **24** is continuous with lumen **25** with a closed end **27** of the shaft **22**. The shaft **22** becomes gradually thinner as it tapers toward the brim **23**.

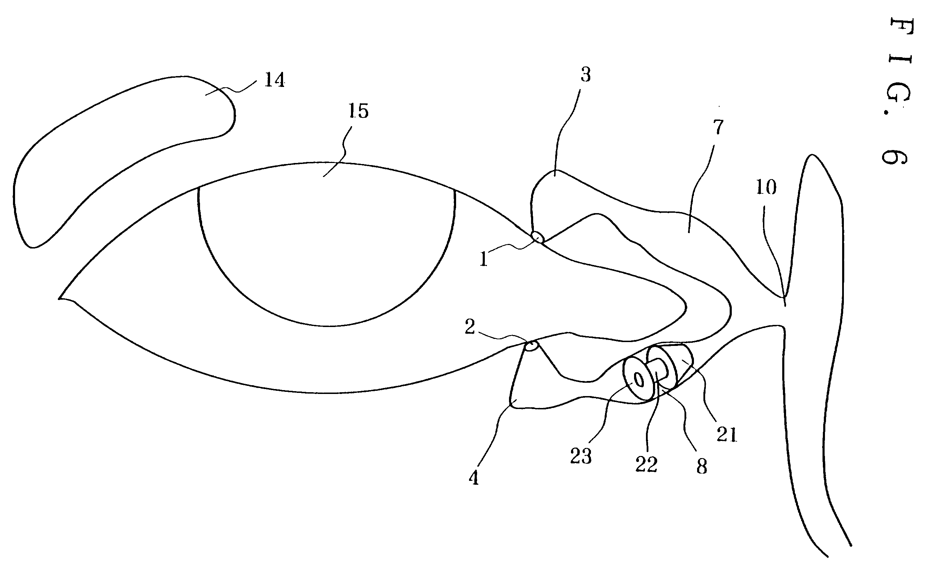

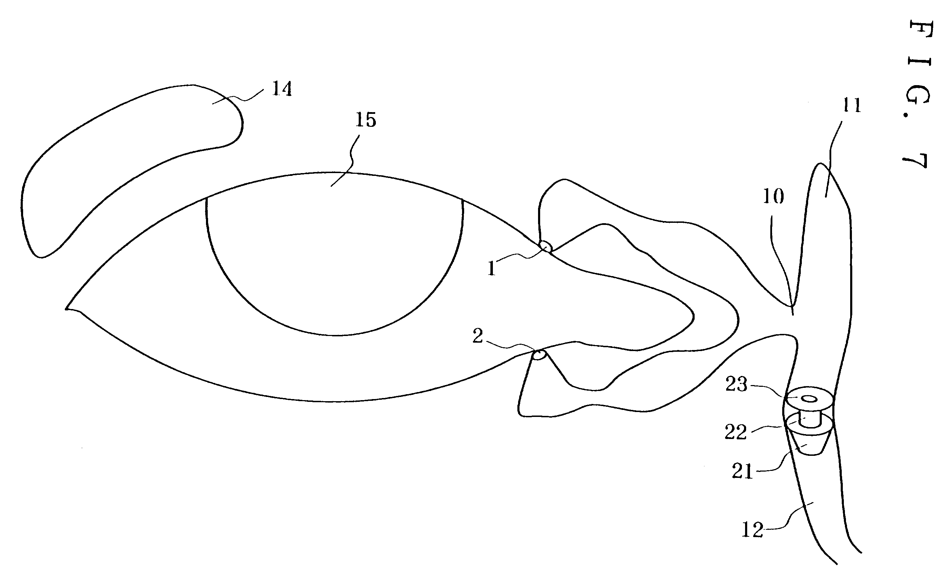

Although corneal disorder is hard to be induced by miniaturization of the punctal plug like this, the punctal plug conversely migrates into the horizontal portion of canaliculus **7, 8** as shown in FIG 6, and as shown in FIG 7 the punctal plug migrates into the lacrimal sac 11 and nasolacrimal duct **12**, resulting in canaliculitis and dacryocystitis which sometimes need surgical interventions (For example, see Rumelt S et al : silicone punctal plug migration resulting in dacryocystitis and canaliculitis. Cornea 16 : 377-399, 1997.).

Let us do a little more explanation in this respect. For dry eye, punctal plugs are inserted into puncta and left in place as shown in FIG 3. But the punctal plug is apt to extrude because of shallow insertion under consideration.

And as shown in FIG 6, 7, the punctal plug migrating into the lacrimal duct is under consideration.

Furthermore, as shown in FIG 2, FIG 4 and FIG 5, the edges of the tip **29** of either punctal plug are angular and sometimes stimulate canaliculus, resulting in the growth of pyogenic granuloma (For example, see Rapoza PA & Ruddat MS : Pyogenic granuloma as a complication of silicone punctal plug. Am J Ophthalmol 113 : 454-455, 1992).

And, stimulation of the tip **29** of punctal plug sometimes induces canalicular abstruction between the vertical portion **3, 4** and horizontal portion of canaliculus (For example, see Fayet B et al : Stenoses canaliculaires compliquant la pose de bouchouns lacrimaux. Incidence et mecanismus, J Fr Ophthalmol 15 : 25-33, 1992.)

Granuloma sometimes pushes the punctal plug out of the puncta.

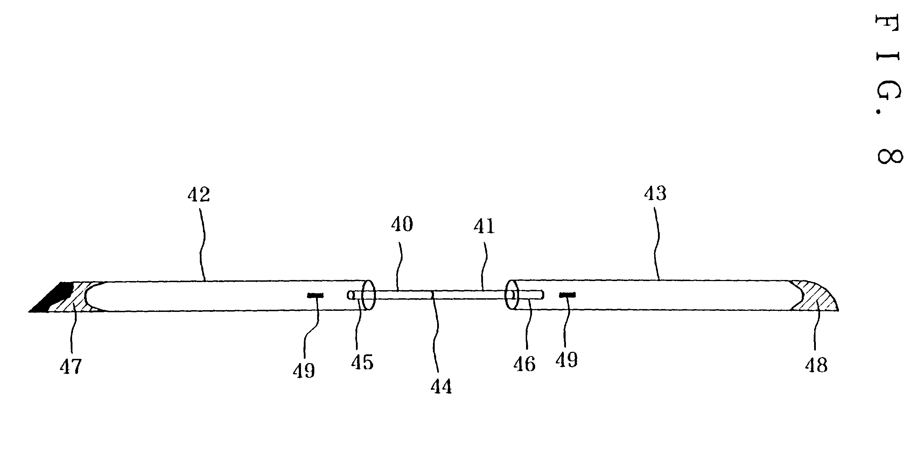

On the other hand, FIG 8∼10 show the hithertofore various nunchaku style silicone tubings which are invented by this inventor. For example, see Patent No.2539325.

Any apparatus for intubation of the lacrimal duct shown in FIG 8∼10 consists of thinner soft tube **40, 41** and thicker tube **42, 43** with certain length, and the ends **47, 48** of the thicker tube are closed end.

Thinner tube **40, 41** exists between two thicker tub**e 42, 43,** and the middle point **44** of the thinner tube **40, 41** is marked.

The thinner soft tube **40, 41** is connected with the thicker tubes **42, 43**. Two millimeter ends of the thinner tube **40, 41** are inserted into the thicker tube **42, 43** for connection. Therefore, the jointed portion **45, 46** are 2mm in length. The tips **47, 48** of the thicker tubes are sharp pointed and closed. For example, 2mm tips of the tube are completely sealed with silastic adhesive, and then diagonally cut to taper the closed ends **47, 48**. Small cuts **49** are applied to the thicker tubes **42, 43** parallel to the tubes **42, 43**.

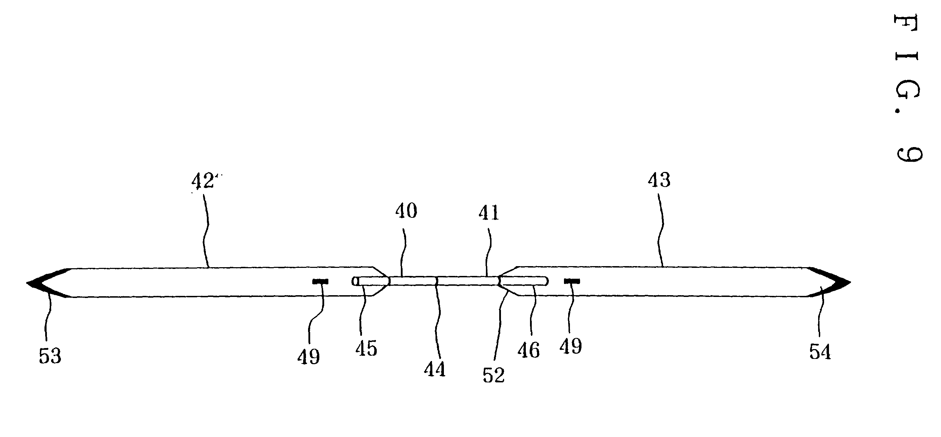

The junctions **45** make steps in the case of FIG 8. As shown in FIG 9∼10, it is possible to make slopes **51** without making any steps.

And in the cases shown in FIG 9∼10, the ends **53, 54** of the thicker tube are in conical shape.

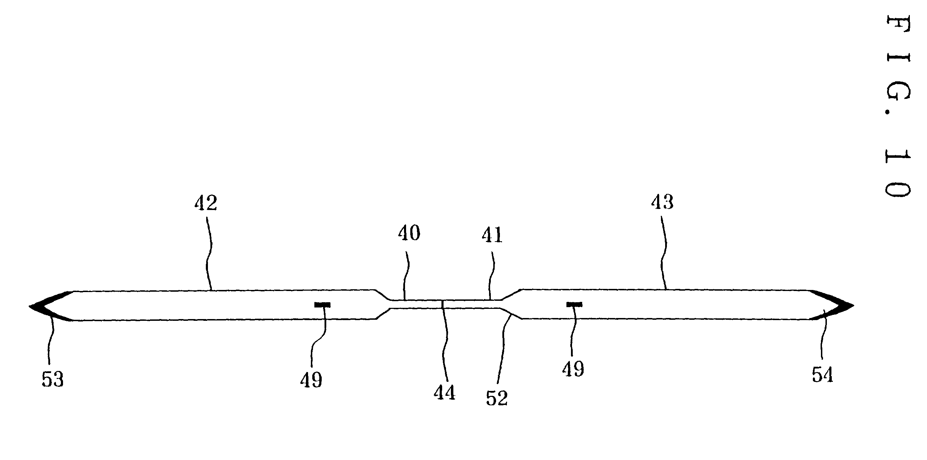

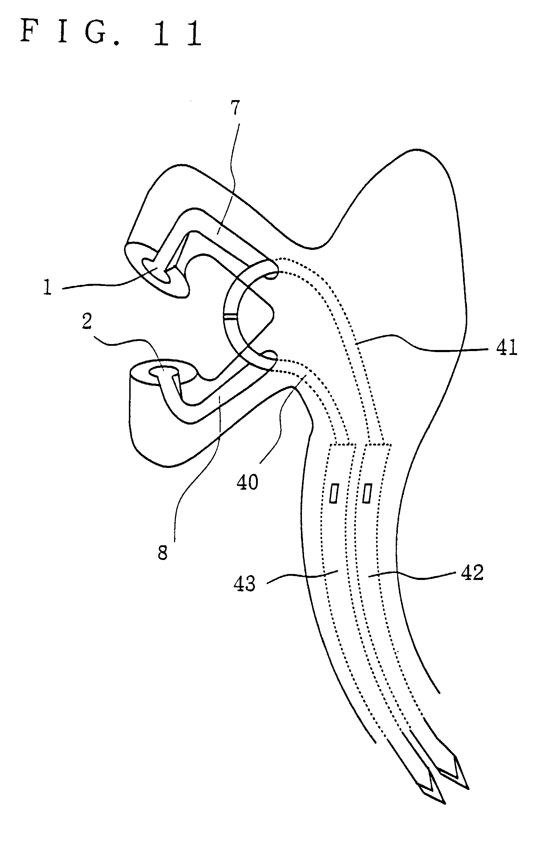

In the cases of FIG 8∼9, it is very rare for the junctions **45** to come off. However, as shown in FIG 10, a one piece tube without any junctions which consists of the thinner tube **40, 41** and thicker tubes **42**, **43** is made from the first.
1) In prior methods of monocanalicular intubation using the half size nunchaku-style silicone tubing shown in FIGS 8∼10 or a prior silicone tube with the same thickness in its total length, it is necessary to fix the tube at the puncta 1, 2 with suturing because they lack the brim.
2) Prior punctal plugs shown in FIGS 2∼7 are anglar, and its stimulation sometimes induce granuloma.
3) Prior punctal plugs shown in FIGS 2∼7, sometimes induce canalicular obstruction between the vertical portion **3, 4** and the horizontal portion **7, 8** of the canaliculus.
4) In prior punctal plugs shown in FIGS 2∼7, the punctal plugs sometimes migrate into the canaliculus, lacrimal sac and nasolacrimal duct because the brim is circular and too small.
5) Prior nunchaku-style silicone tubings shown in FIGS 8∼10 are sometimes difficult to insert from the puncta **1, 2** because its closed ends are not so sharp pointed.
6) Prior punctal plugs shown in FIGS 2∼7, sometimes come off because of its shallow insertion.
7) Dry eye symptoms are sometimes aggravated in patients with both dry eye and dacryocystitis, after intubation using prior tubes shown in FIGS 8∼10 and/or dacryocystothinostomy.
8) Punctal plugs shown in FIGS 2∼7, are not stable.
9) Tubes with the same thickness in its total length are not stable even if the brim is attached to them.
10) Prior tubes shown in FIGS 8∼10 sometimes induce slitting of the puncta **1, 2** and canaliculi **3∼8**, as shown in FIG 11.

### SUMMARY OF THE INVENTION

It is an object of the present invention to provide an apparatus for intubation of the lacrimal duct which is stable in the lacrimal duct, can be easily inserted into the lacrimal duct and be easily removed, is not in danger of extrusion after leaving in place and growth of granuloma.

It is another object of this invention to provide an apparatus for intubation of the lacrimal duct which can be used for reconstruction of lacrimal duct obstructions.

One apparatus according to this invention is an apparatus for intubation of the lacrimal duct which is characterized by the presence of, a thinner tube or rod, a prescribed length of thicker tube which is joined with one end of the thinner tube or rod, and a stopper which is joined with the other end of the thinner tube or rod in apparatus for intubation of lacrimal duct inserted into the lacrimal duct.

Other apparatus according to this invention is an apparatus for intubation of lacrimal duct which is characterized by presence of a prescribed length of thicker tube and a stopper joined with the posterior end of the thicker tube.

Other apparatus according to this invention is an apparatus for intubation of lacrimal duct which is characterized by the fact that said stopper is a punctal plug, brim, ring and so on.

The inventor has studied keenly for many years for the treatment of lacrimal duct obstruction and dry eye apparatus for intubation of lacrimal duct which can be used easily with decrease pain to patients, can be quickly and correctly inserted into the lacrimal duct, is not easily dislocated during intubation period, and can be easily removed after accomplishment of treatment.

This invention is to provide a more improved apparatus for intubation based on the apparatus which has been developed until now. This invention especially improves the stableness of the apparatus for intubation in the lacrimal duct.

In the present invention, an apparatus for intubation of the lacrimal duct includes a stopper. As a stopper, a punctal plug, brim, ring and other things are used.

Apparatus for intubation according to the present invention consists of a prescribed length of thinner tube or rod, prescribed length of thicker tube which is connected with one end of thinner tube or rod, and punctal plug which is connected with the other end of thinner tube or rod.

Other apparatus for intubation according to the present invention omits the thinner tube or rod from said embodiment, consists of a prescribed length of the thicker tube and the punctal plug which connected with the posterior end of the thicker tube.

Other apparatus for intubation according to the present invention wherein the punctal plug of said apparatus for intubation is changed into a brim, consists of a prescribed length of thinner tube or rod, a prescribed length of thicker tube which is connected wih one end of the thinner tube or rod, and a brim which connected with the other end of the thinner tube or rod.

Other apparatus for intubation according to the present invention, wherein the punctal plug of said apparatus for intubation is changed into a stopper, consists of a prescribed length of thinner tube or rod, a prescribed length of thicker tube which connected with one end of the thinner tube a rod and the stopper which is connected with the other end of the thinner tube or rod.

As stated above, use of punctal plug, brim and ring as a stopper brings about a great effect which each cannot be gained by prior arts.

In any said apparatus for intubation it is preferable that the tip of the thicker tube is closed.

Furthermore, in a preferable apparatus for intubation according to the present invention, its total length is 15∼60mm including tube and punctal plug, punctal plug is 1.5∼2.5mm in length, thicker tube is 10∼59mm in length and thinner tube is 1∼5mm in length.

In other preferable apparatus for intubation according to the present invention, a thicker hard tube is connected with a punctal plug via a thinner soft tube.

As a further other mode different from this, the thicker hard tube is directly connected with the punctal plug without intervention of the thinner soft tube to constitute an apparatus for intubation.

In yet another further other mode of the present invention, various nunchaku-style silicone tubings are fixated with various punctal plugs with silastic adhesive to be able to constitute an apparatus for intubation.

In another further other mode of the present invention, central segment is flexible, both ends of it are fixated to the punctal plug and tube with silicone adhesive, the punctal plug and tube are thicker and harder, and the central segment constitutes has constitution to be able to pass through with forming a curve the boundary portion between the vertical and horizontal portions of canaliculus.

And the thicker tube which is used in this invention has a closed end and a small cut is applied to the part of the tube for probe to insert. This makes for the apparatus to be inserted into the lacrimal duct easily.

In the punctal plug used in this invention, the brim preferably 1.5∼4.5mm in diameter prevents the punctal plug from migrating into the lacrimal duct, and simultaneously prevents tears from flowing into the puncta.

The apparatus for intubation of this invention has a great stability in the lacrimal duct compared with conventional punctal plug and it is very rare to extrude.

### BRIEF DESCRIPTION OF THE DRAWINGS

FIG.1 is a schematic diagram of the lacrimal duct.
FIG.2 is a schematic diagram of the prior art.
FIG.3 is a schematic diagram showing how to use the punctal plug in FIG.2.
FIG.4(A) is a schematic diagram showing a conventional other plug.
FIG.4(B) is the bottom view of it.
FIG.4(C) is the mid-cross-sectional view of it.
FIG.5(A) is a schematic diagram showing conventional other plug.
FIG.5(B) is the bottom view of it.
FIG.5(C) is the mid-cross-sectional view of it.
FIG.6 is a diagram showing a failure when the plug in FIG.2 is used.
FIG.7is a diagram showing another failure when the plug in FIG.2 is used.
FIG.8 is a diagram showing a conventional nunchaku-style silicone tubing.
FIG.9 is a diagram showing other conventional nunchaku-style silicone tubing.
FIG.10 is an explanatory diagram showing still other conventional nunchaku-style silicone tubing.
FIG.11 is an explanatory diagram showing a failure in a conventional nunchaku-style silicone tubing.
FIG.12 is a schematic diagram showing the apparatus for intubation in the present invention.
FIG.13 is a schematic diagram showing other apparatus for intubation in the present invention.
FIG.14 is a schematic diagram showing other apparatus for intubation in the present invention.
FIG.15 is a schematic diagram showing other apparatus for intubation in the present invention.
FIG.16 is a schematic diagram showing other apparatus for intubation in the present invention.
FIG.17 is a schematic diagram showing other apparatus for intubation in the present invention.
FIG.18 is a schematic diagram showing other apparatus for intubation in the present invention.
FIG.19 is a schematic diagram showing other apparatus for intubation.
FIG.20 is a cross sectional view showing other apparatus for intubation.

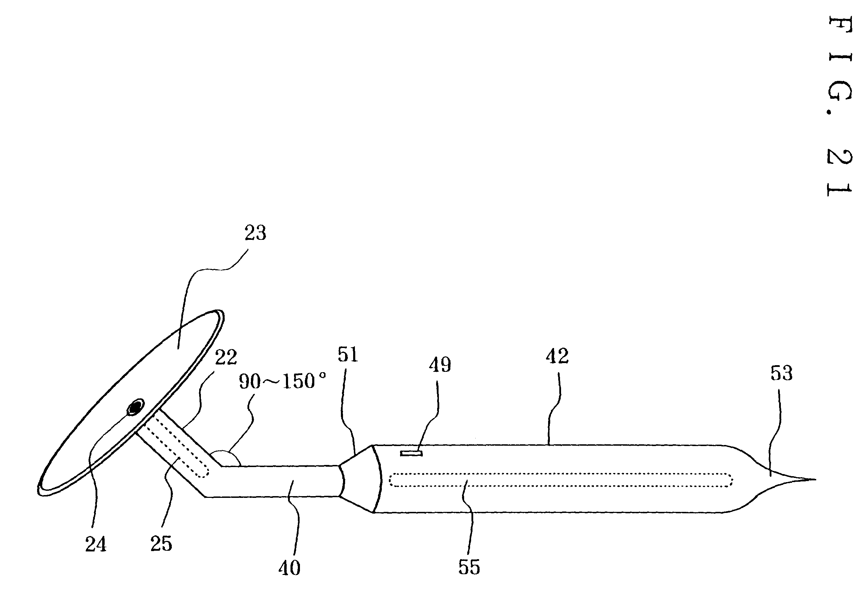

FIG.21 is a perspective view showing the apparatus for intubation in Fig.20.
FIG.22 is a cross sectional view showing other apparatus for intubation.

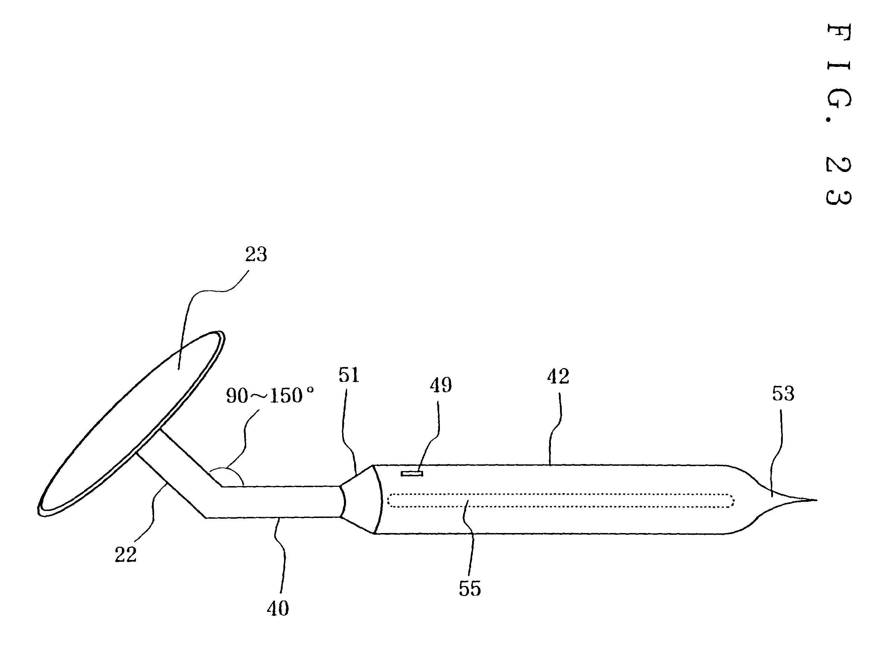

FIG.23 is a perspective view showing the apparatus for intubation in Fig.22.
FIG.24 is a cross sectional view showing other apparatus for intubation.

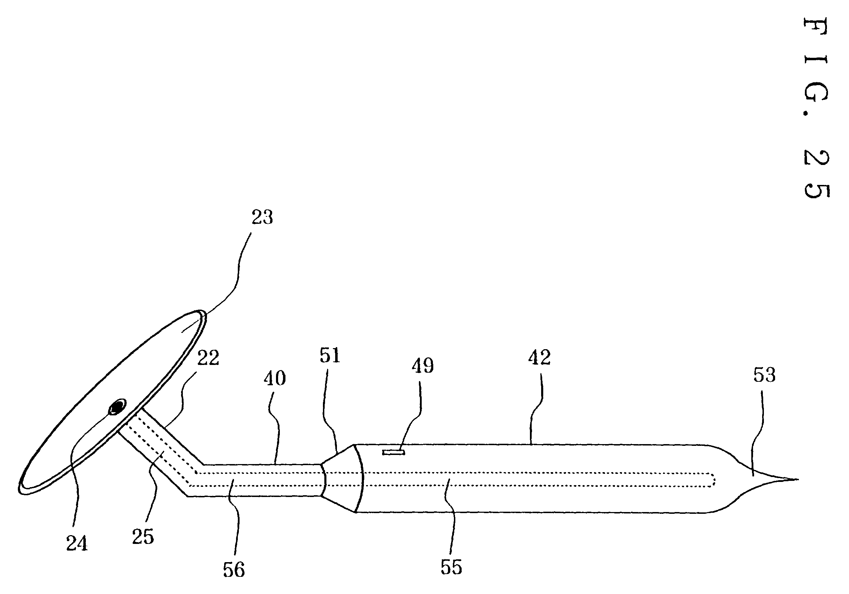

FIG.25 is a perspective view showing the apparatus for intubation in Fig.24.
FIG.26 is a perspective view showing the method of insertion of the apparatus in Figs.17∼25.
FIG.27 is a diagram showing monocanalicular intubation method in Figs.17∼25.
FIG.28 is a diagram showing other monocanalicular intubation method in FIGS 17∼25.
FIG.29 is a diagram showing bicanalicular intubation method in Figs.17∼25.
FIG.30 is a sectional view showing other apparatus for intubation in the present invention.

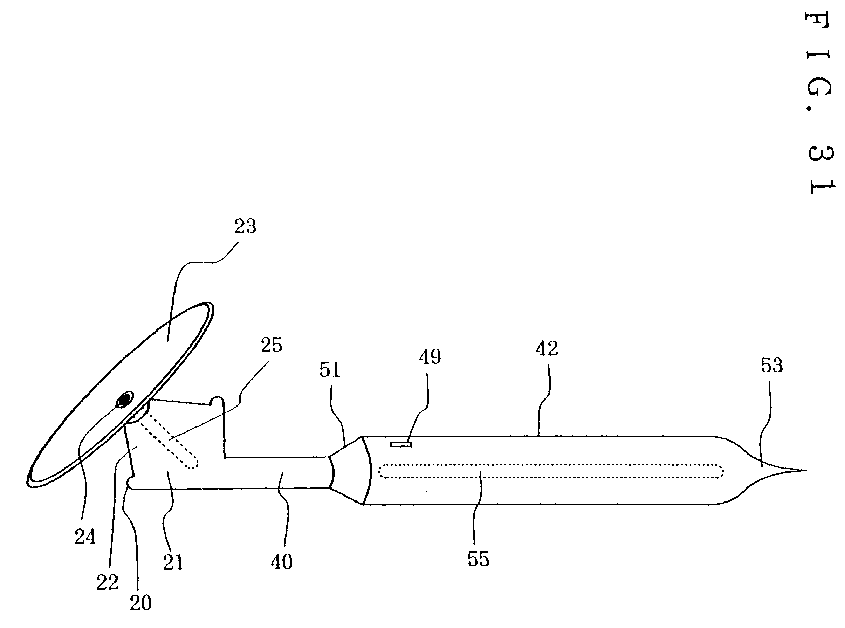

FIG.31 is a perspective view showing the apparatus for intubation in Fig.30.
FIG.32 is an explanatory diagram showing the insertion method of the apparatus for intubation in Figs.30∼31.
FIG.33 is an explanatory diagram showing a monocanalicular intubation method using the apparatus for intubation in Figs.30∼31.
FIG.34 is an explanatory diagram showing other monocanalicular intubation method using the apparatus for intubation in Figs.30∼31.
FIG.35 is an explanatory diagram showing bicanalicular silicone intubation method using the apparatus for intubation in Figs.30∼31.
FIG.36 is a cross sectional view showing other apparatus for intubation in present invention.

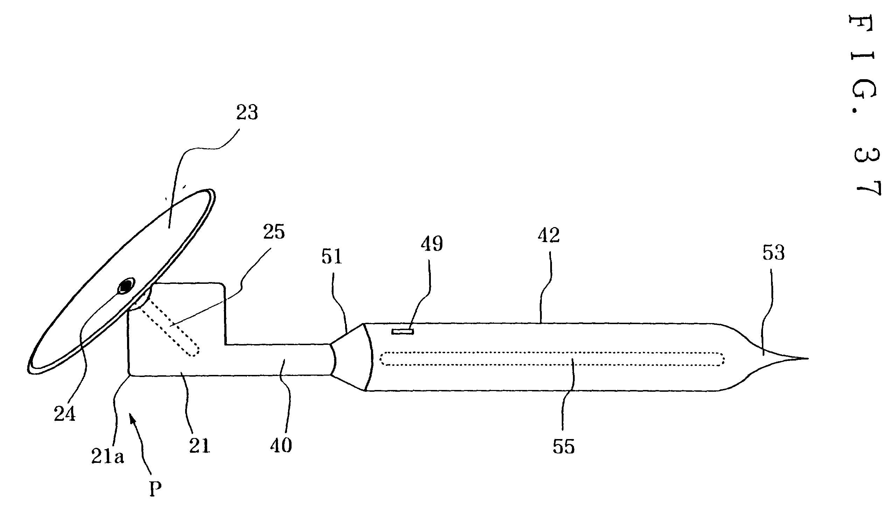

FIG.37 is a perspective view showing the apparatus for intubation in Fig.36.
FIG.38 is an explanatory diagram showing the method of insertion of the apparatus for intubation in Figs.36∼37.
FIG.39 is an explanatory diagram showing monocanalicular silicone intubation method in Figs.36∼37.
FIG.40 is an explanatory diagram showing other monocanalicular silicone intubation method using the apparatus for intubation in Figs.36∼37.
FIG.41 is an explanatory diagram showing bicanalicular intubation using the apparatus for intubation in Figs.36∼37.
FIG.42 is a perspective view showing still other apparatus for intubation in the present invention.

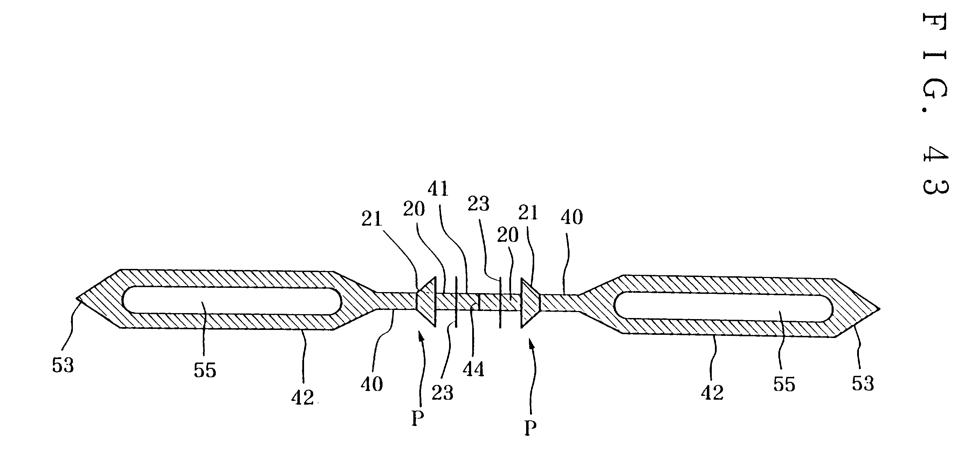

FIG.43 is a mid-cross sectional view showing the apparatus for intubation in Fig.42.
FIG.44 is an explanatory diagram showing the method of placement of the apparatus for intubation in Figs.42∼43.

### DETAILED DESCRIPTION OF THE EMBODIMENTS

The embodiments of this invention will be explained, referring to figures.

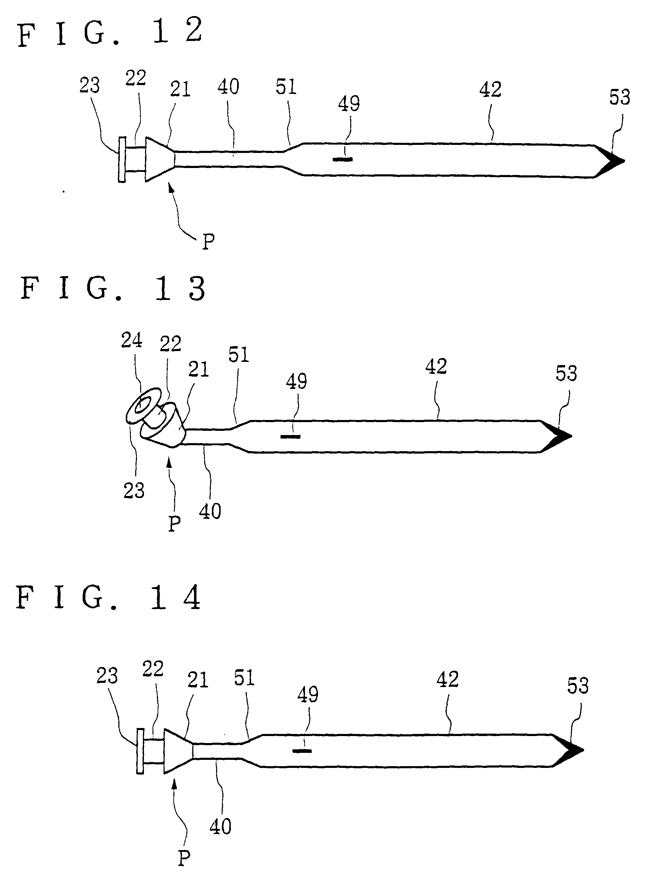

FIG 12∼14 show three different embodiments of this invention.

In the embodiments of FIG 12∼14, the punctal plug **P** is attached to an end of the thinner soft tube **40**, 1∼15mm in length, with silicone adhesive. The posterior end of the thicker hard tube **42** is the other end of the thinner tube **40**. The tip of the thicker tube **42** is closed in a conical shape.

The punctal plug P consists of the tip **21** which shape is the frustum of the circular cone, brim **23** which shape is circular elliptical and its whole body is a single piece. In the center of the brim **23**, a hole **24** is formed. The hole **24** is connected with the lumen of the shaft **22**, and the lumen of the tip **21**, and connected with the inner space of the thinner tube **40** and the inner space of the thicker tube **42** with a closed end **53**.

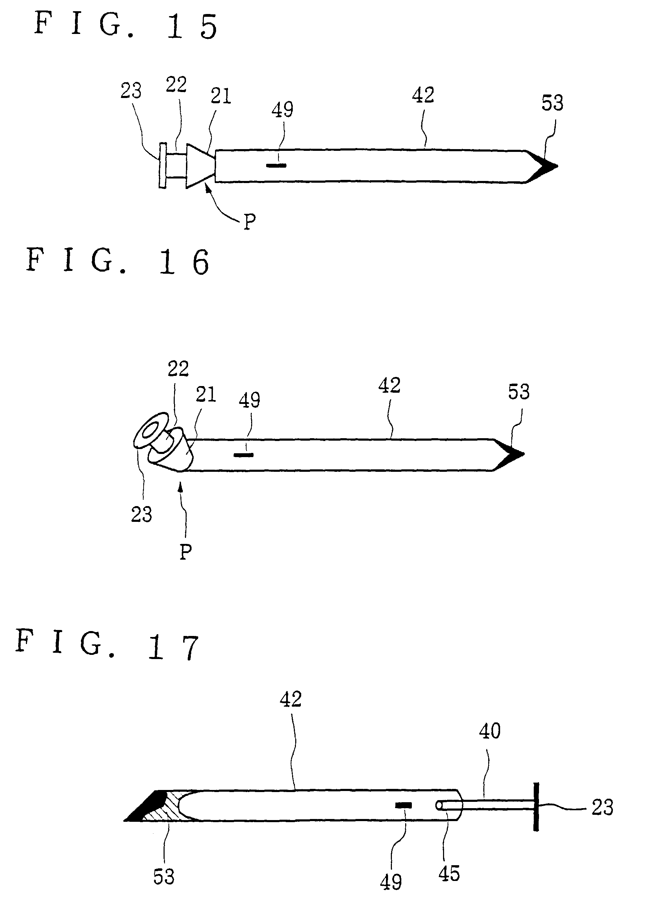

In embodiments of FIG 12 and FIG 14, the axis of the thinner tube **40** is coincident with the axis of the thicker tube **42** and the axis of the plug. Whereas, although in embodiment of FIG 13, the axis of the thinner tube **40** is coincident with the axis of the thicker tube **42**, the axis of the plug is not coincident with the axis of the thinner tube **40** and the axis of the thicker tube **42** to be formed at a prescribed angle (for example 90∼150° ).

Although the plug without hole **24** can be used and a rod instead of thinner tube **40** can be used in embodiment of FIGS 12∼14, the hole **24** of the plug P is connected with the closed end **53** via the lumen of the shaft **22**, the inner space of the thinner tube **40**, the inner space of the thicker tube **42** and the lumen of the tip **21**. Its form will be explained.

Regarding Materials for tube **40, 42** and punctal plug P, it is important to select one which is substantially unstimulating and non-toxic to the tissue of the eye and a living body. From this point of view, silicone is appropriate because its safety is already established as a apparatus for treatment of lacrimal duct obstruction. Above all, the combination of silicone tube 0.9∼1.2mm OD and 0.5∼0.7mm OD and 0.3∼0.5mm ID is especially preferably used. Regarding punctal plug P, silicone punctal plugs are preferable as shown in FIG 12∼14.

This composition will be concretely explained as follows. As shown in FIG 12∼14, the right end of the thinner soft tube (0.5∼0.7mm OD, 0.3∼0.5mm ID, 2∼20mm in length, **40** is connected with the left end of the thicker tube (0.9∼1.2mm OD, 0.5∼0.7mm ID, 5∼50mm in length) **42**. The tip **53** of the thicker tube **42** is sharp pointed and closed. For example, 2mm tip of the thicker tube **42** is completely sealed with silastic adhesive, and then diagonally cut to taper the closed end **53**. Small cuts 0.5mm in length **49** for probe 0.4mm in length to insert is applied to the thicker tube **42** parallel to the tub**e 42**. If the small cut is applied perpendicularly to the tube, the tube may be broken during operation. The preferable position of the small cut is 10 to 45mm from the tip of the thicker tube **42**. The tube **42** can be easily inserted into the lacrimal duct by inserting the probe (not illustrated) from the small cut **49**. Making corresponding to the position of the small cut **49** make it easier to discover the small cut.

Preferable total length of the apparatus of the present invention is as follows.

Total length of 40∼60mm apparatus are appropriate for adult-nasolacrimal duct obstruction and total length of 30∼50mm apparatus for child-nasolacrimal duct obstruction. Total length of 10∼60mm apparatus are useful for reconstruction of canalicular obstruction and for using as a punctal plug.

The length and thickness of the silicone tube depends on the length and size of the inner space of the individual lacrimal duct. The most used one being 51.7mm in total length consists of the thinner tube **40** 0.64mm in thickness, 10mm in length, the thicker tube **42** 0.94mm in thickness, 40mm in length, and the punctal plug 1.7mm in total length.

In order to be stable in the lacrimal duct, it is important that the thinner tube is softer. As long as it is thinner and softer, for example the soft rod without an opening 0.5∼0.7mm in diameter can be used instead of the thinner tube **40**.

It is better to make a slope **51** without making any steps at the junction.

If the tip **53** of the thicker tube is sharp pointed in a conical shape, it is more easily inserted from the lacrimal puncta.

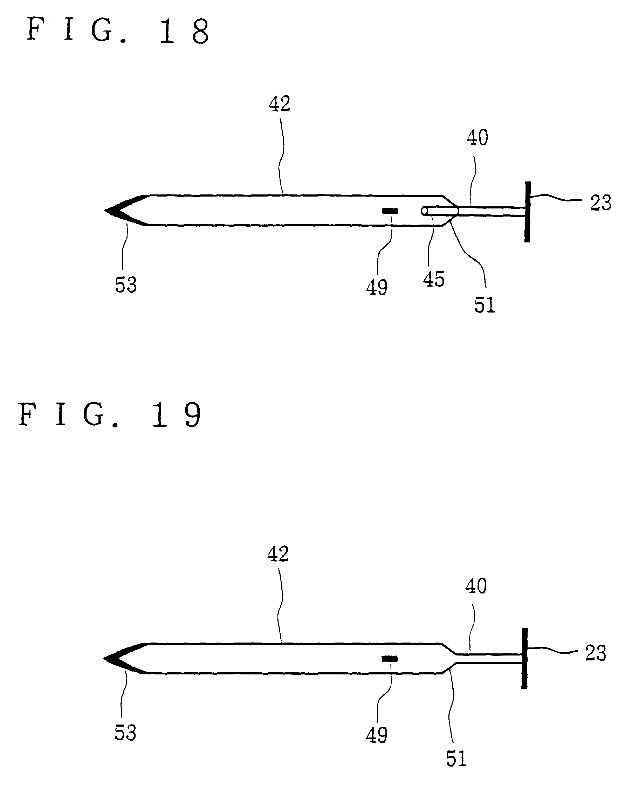

The embodiment in FIG 12∼14 are one piece without junctions which consists of the thinner tube **40** and the thicker tube **42**. As shown in FIG 17∼18, it can be made by 2mm end of the thinner tube inserted into the thicker tube for connection and fixation using silicone glue. In the embodiment in FIG 18, the junction between the thinner tube **40** and the thicker tube **42** has no steps, and in the case of FIG 17 the junction between the thinner tube **40** and the thicker tub**e 42** is a step-like junction.

Although it is not illusrated, it is better for the tube to be equipped with the probe from the first.

Constitutions of the embodiment in FIG 8∼11 suit with bicanalicular intubation in which a tube **40, 42, 41, 43** is introduced into the lacrimal duct from the upper and lower puncta. Whereas, constitutions of the embodiment in FIG 12∼16 suit with monocanalicular intubation in which the apparatus for intubation is inserted into the lacrimal duct from the upper puctum only (or the lower punctum only).

In embodiment in FIG 15 and FIG 16, the punctal plug P is attached to the posteinor end of the thicker hard tube **42** with silicone glue without using a thinner soft tube. The tip **53** of the thicker tube is in conical shape and closed. Punctal plug P is one piece which consists of the tip **21** which is a shape of the frustum of the circular cone, the shaft **22** which is tubular, and the bri**m 23** which is circular. In the center of the brim **23**, the hole **24** is applied. The hole **24** is connected with the lumen of the shaft **22** and the lumen of the tip **21** which is connected with the inner space of the thicker tube **42** with a closed end **53**.

The tube **42** can be easily inserted into the lacrimal duct by insertion of the probe (not illustrated) from the small cut **49** applied to the thicker tube **42**.

The embodiments of the constitution in FIG 15∼16 is superior for the treatment of the lacrimal duct obstruction. As shown in FIG 15∼16, only the thicker tube **42** is used and the thinner tube **40** is abbreviated. The tip **53** of the thicker tube **42** is in a conical shape and closed. When this is inserted into the lacrimal duct from the upper punctum **1**, the tube **42** is pushed into the lacrimal duct by the probe **61** (mentioned later) which is inserted into the tube **42** till the tip **53**. After insertion of the tub**e 42**, the probe **61** is removed.

In the embodiment in FIG 15, the axis of the thicker tube **42** is coincident with the axis of the plug P. Whereas, in the embodiment in FIG 16, the axis of the plug P is not coincident with the axis of the thicker tube **42** to form at a prescribed angle (for example 90∼150° ).

FIGS 17∼19 show other embodiments in this invention besides.

In the embodiment in FIG 17, the brim **23** is joined with one end of the thinner soft rod **40** 5∼20mm in length with silicone glue. The brim **23** is formed rather hard silicone.

In the embodiment of FIG 18, the brim **23** is joined with one end of the thinner soft rod **40** 5∼20mm in length, The brim **23** is formed by rather hard silicone. The posterior end of the thicker hard tube **42** is joined with the other end of the thinner tube **40**. The ti**p 53** of the thicker tube **42** is in conical shape and closed. The thinner ro**d 40** and the thicker tube **42** are sparately made and 2mm end of the thinner rod is inserted into the thicker tube for connection with silicone glue. This embodiment in FIG 18 has not any step in the junction between the thinner rod **40** and the thicker tube **42**.

In the embodiment in FIG 19, the brim **23** is joined with one end of the thinner soft tube **40** with silicone glue. The brim **23** is formed by rather hard silicone. The posterior end of the hard tube **42** is joined with the other end of the thinner tube **40**. The ti**p 53** of the thicker tube **40** is in conical shape and closed. The thinner tube **40** is joined with the thicker tube **42** with the shape of one body. In the embodiment in FIG 19, the junction between the thinner tube **40** and the thicker tube **42** has not any step.

In the embodiment in FIG 17∼19, the tube **42** can be inserted into the lacrimal duct easily by a probe (not illustrated) which inserted into the small cut **49** of the thicker tube **42**.

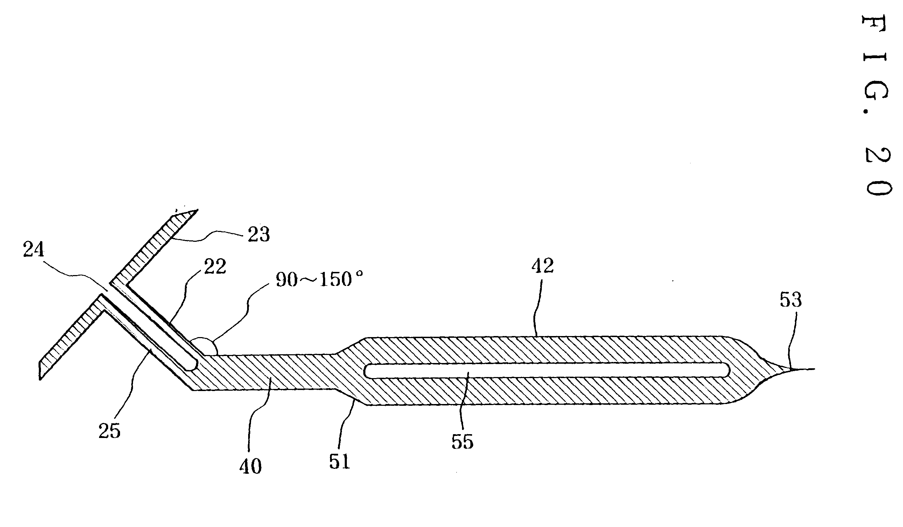

FIG 20∼21 show other embodiments in the present invention besides. The thinner soft rod consists of the segment **22** 0.5mm OD and 2∼2.5mm in length, and the segment **40** 0.5mm OD and 3∼20mm in length, and the segments **22, 40** form at angle of 90∼150 degrees. The brim **23** is joined with an end of the thinner soft rod with silicone glue. The brim **23** is circular, elliptic and others in shape, and formed by hard silicone. The posterior end of the thicker hard tube **42** 30∼40mm in length is joined with the other end of the thinner rod to form a shape of single body. The tip **53** of the thicker tube **42** is in conical shape and closed. The lumen **25** formed in the thinner rod **22** is connected with the hole **24** of the brim **23**. The lumen **25** extends into the thinner rod till the corner which exists halfway in the thinner rod. The thicker tube **42** 1.0∼1.2mm OD and 0.5mm ID has the inner space **55**. The junction between the thinner rod **40** and the thicker tube **42** is a slope **51** not to make a step. And, in this embodiment, the axis of the brim **23** is set to the axis of the thicker tube **42** at a prescribed angle (for example 90∼150 degrees).

The embodiments in FIG 20∼21, the tube **42** can be easily inserted into the lacrimal duct by the probe (not illustrated) which is inserted into the inner space **55** from the small cut **49** applied to the thicker tube **42**.

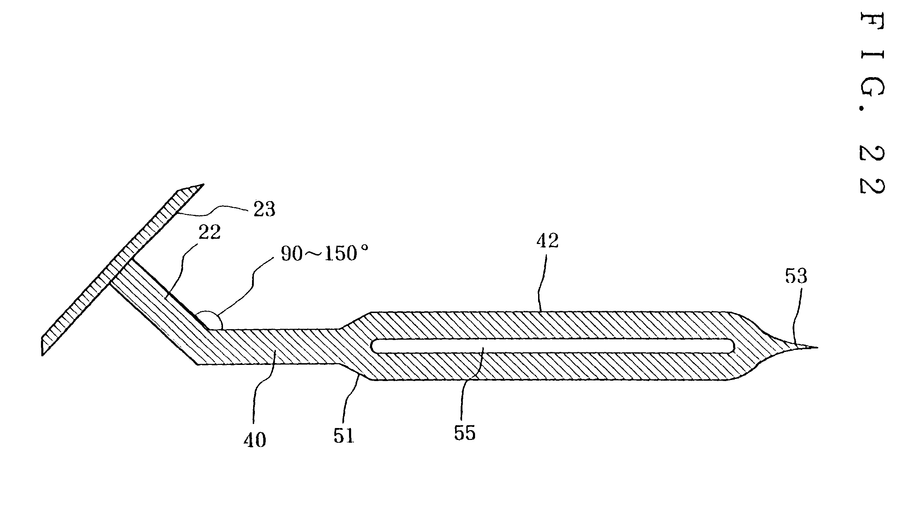

FIG 22∼23 show still other embodiment of the present invention besides. The thinner soft rod without a lumen consists of the segment **22** 0.5mm OD, 1.5∼2.5mm in length and the segment **40** 0.5mm OD, 3∼20mm in length, and at an angle of 90∼150 degrees between these segments **22, 40**. The brim **23** is joined with an end of such a thinner soft rod with silicone glue. The brim **23** is circular elliptical and in other shapes and is formed by hard silicone. The posterior end of the thicker hard tube **42** 30∼40mm in length is joined with the other end of the thinner rod to be a single body. The tip **53** of the thicker tube **42** is sharp pointed and has a closed end. The thicker tube 1.0∼1.2mm OD and 0.5mm ID has the inner space **55** which extends to the closed end. The junction between the thinner rod **40** and the thicker tube **42** is a slope **51** not to make a step. And, in this embodiment, the axis of the brim **23** is set at a prescribed angle (for example 90∼150 degrees) to the axis of the thicker tube.

In the embodiments shown in FIGS 20∼21, the tube **42**, can be easily inserted into the lacrimal duct by the probe (not illustrated) which is inserted from the small cut **49** applied to the thicker tube **42**.

FIGS 22∼23 show still other embodiments according to the present invention. The thinner soft rod without any opening, consists of the segment **22** 0.5mm OD and 1.5∼2.5mm in length and the segment **40** 0.5mm OD and 3∼20mm in length, these segments **22, 40** form an angle of 90∼150 degrees. The brim **23** is attached to an end of such a thinner soft rod with silicone glue. The brim **23** is circular elliptic and in other forms, and manufactured from hard silicone. With the other end of the thinner rod, the posterior end of the thicker hard tube is connected in the form of one body. The tip **53** of the thicker tube is sharp pointed and closed. In the thicker tube **42** 1.0∼1.2mm OD and 0.5mm ID, the inner space **55** extends to the closed end. The junction between the thinner rod **40** and the thicker tube **42** has the slope **51** without making any step. And, in this embodiment, the axis of the brim **23** is at a prescribed angle (for example 90∼150 degrees) to the axis of the thicker tube **42**.

In the embodiments shown in FIGS 22∼23, the tube **42** can be easily inserted into the lacrimal duct by the probe (not illustrated) which inserted from the small cut **49** which applied to the thicker tube **42**.

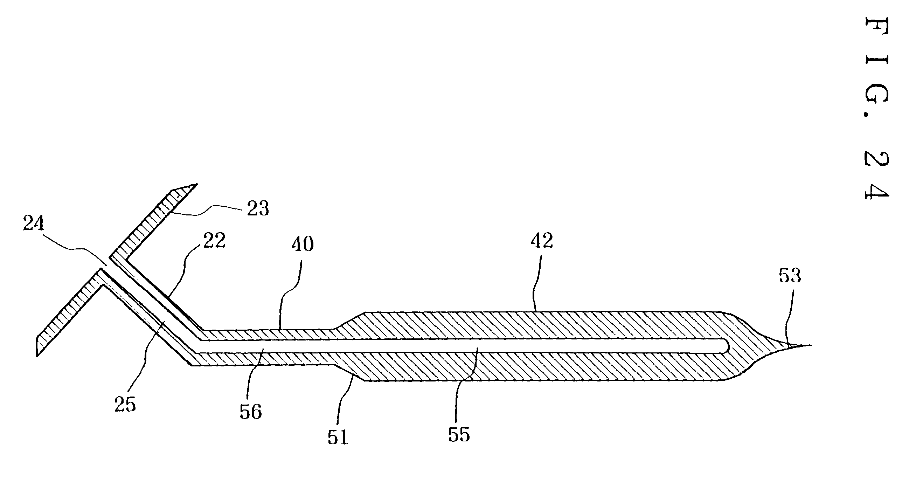

FIGS 24∼25 show still other embodiments according to the present invention. The thinner soft rod consists of the segment **22** 0.5mm OD and 2∼2.5mm in length and the segment **40** and these segments **22, 40** form an angle of 90∼150 degrees. The brim **23** is attached to an end of the thinner soft rod with silicone glue. The brim **23** is circular, elliptical and in other forms, and manufactured from hard silicone. The posterior end of the thicker hard tube **42** 30∼40mm in length is connected with the other end of the thinner rod forming one body. The tip **53** of the thicker tube **42** is sharp pointed and closed. The lumen **25** created in the segment of the thinner rod **22** is connected with the hole **24** of the brim **23**. The lumen **25** extends in the total length of the thinner rod. The thicker tube **42** 1.0∼1.2mm OD and 0.5mm ID has the lumen **55**. The lumen **25** of the thinner tube **40** is connected with the lumen **55**. The junction is a slope **51** without making any steps. And, in this embodiment, the axis of the brim **23** is at a prescribed angle (for example 90∼150 degrees) to the axis of the thicker tube **42**.

In the embodiment shown in FIGS 24∼25, the thicker tube can be easily inserted into the lacrimal duct by the probe (not illustrated) which inserted into the small cut **49** applied to the thicker tube **42**.

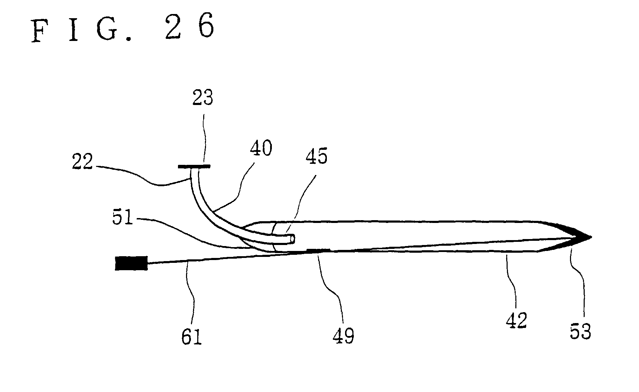

FIG 26 shows still another embodiment according to the present invention. The brim **23** is attached to an end of thinner soft rod **40** with silicone glue. The brim **23** is circular, elliptical and in other shapes and made of hard silicone. Into the posterior end of the thicker tube, the thinner rod **40** is inserted to join them. The segment of the thinner rod **22** forms a curve from junction **45** to the brim **23**. The junction **45** is formed so as to be a slope without making any step. And in this embodiment, the axis of the brim **23** is at a prescribed angle (for example, 90∼150 degrees) to the axis of the thicker tube **42**.

The way of closing the embodiment shown in FIG 26 is substantially the same as the above mentioned embodiments shown in FIGS 20∼25, and the tube **42** can be easily pushed into the lacrimal duct by the probe **61** which is inserted into the inner space **55** from the small cut **49** applied in the thicker tube **42**.

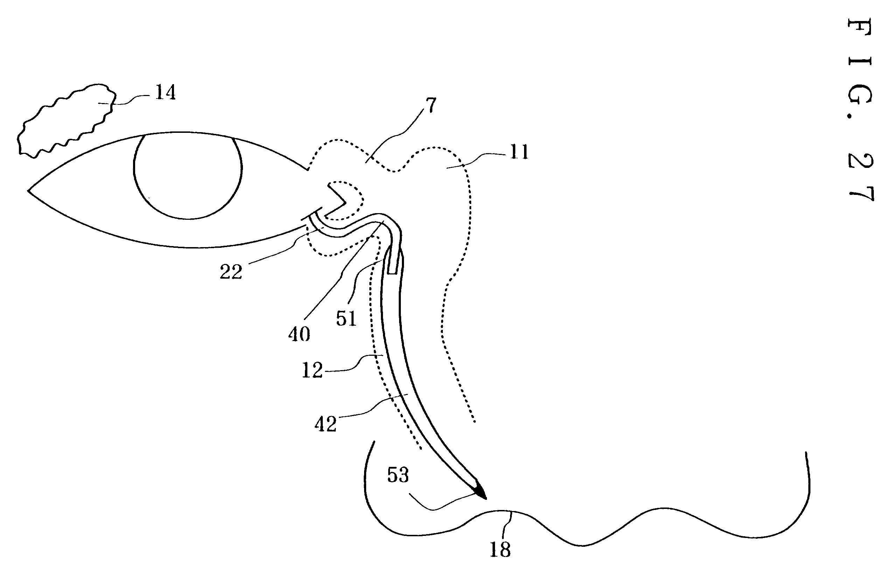

FIG 27 shows one of the post-operative state of placement of the apparatus for intubation in the present invention inserted from the lower canaliculus in the embodiments in FIGS 20∼26.

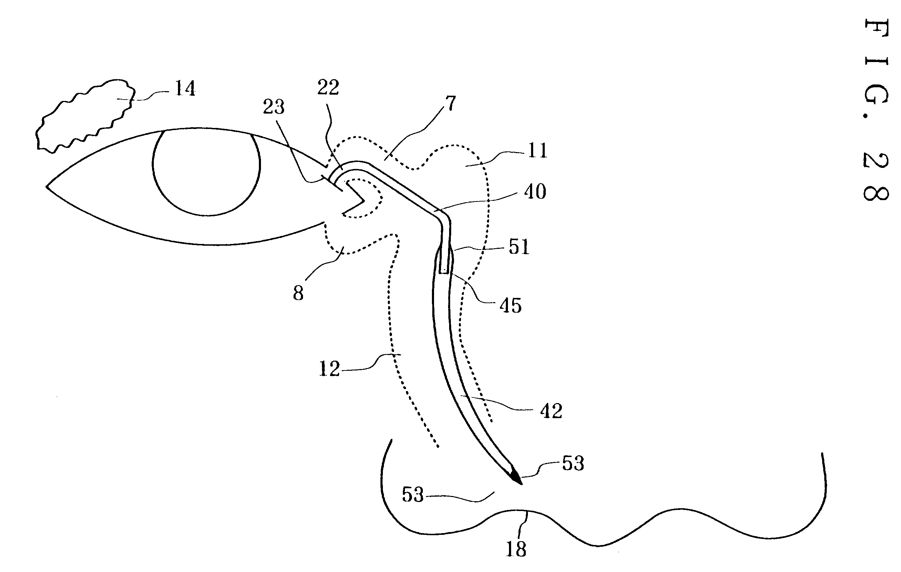

FIG 28 shows one of the post-operative state of placement of the apparatus for intubation in the present invention inserted from the upper canaliculus in the embodiments in FIGS 20∼26.

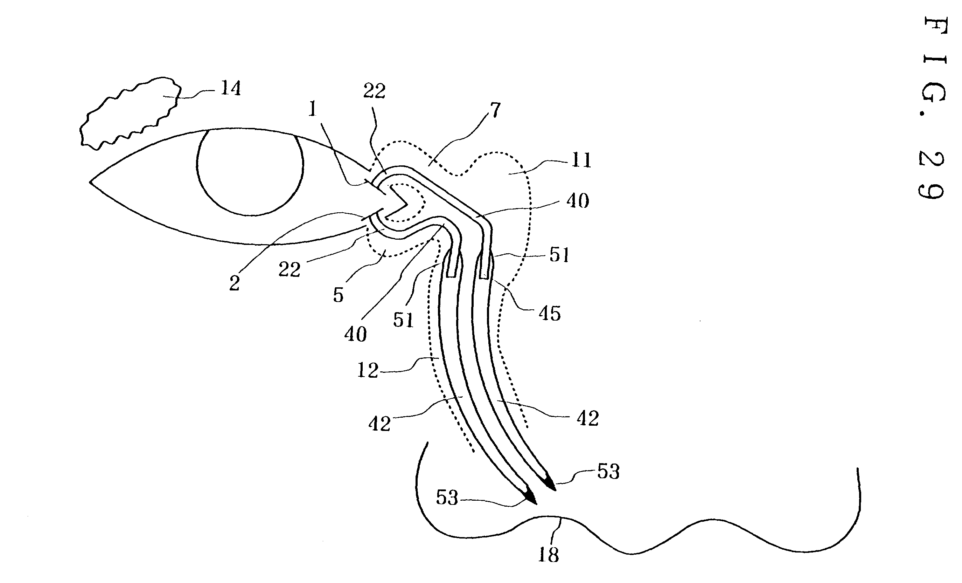

FIG 29 shows one of the post-operative state of placement of the apparatus for intubation in the present invention inserted from the upper and lower canaliculi as to the embodiments in FIGS 20∼26. This is the most suitable placement for the treatment of dry eye.

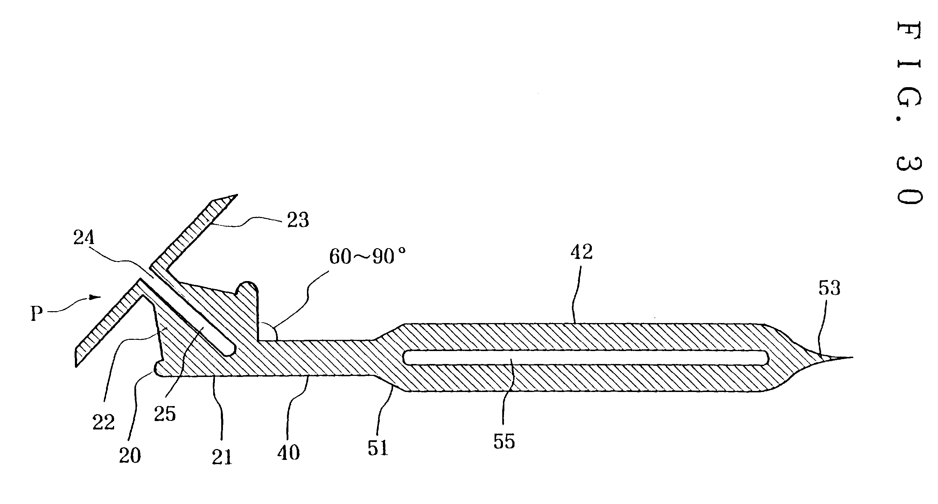

FIGS 30∼31 show still other embodiment in the present invention. The punctal plug P is attached to an end of the thinner soft rod **40**. The punctal plug P consists of the tip **21**, intermediate portion **22** and the brim **23**. The edge **20** is round not to induce granulation. The brim **23** is circular, elliptical and in other shapes and made of hard silicone. The posterior end of the thicker hard tube 30∼40mm in length in connected with the other end of the thinner rod to be a form of one body. The tip **53** of the thicker tube **42** is sharp pointed and closed. The lumen **25** created in plug P is connected with the hole **24**. The thicker tube **42** has the inner shape **55** and its outer diameter is 1.0∼1.2mm, and inner diameter is 0.5mm. The junction is slope not to make any steps. And, in these embodiments also, the axis of the brim **23** is at a prescribed angle (for example 90∼150 degrees) to the axis of the thicker tube **42**.

In the embodiments in FIGS 30∼31 also, the thicker tube **42** can be easily inserted into the lacrimed duct by the probe **61** which is inserted into the inner space **55** from the small cut **49** of the thicker tube **42**.

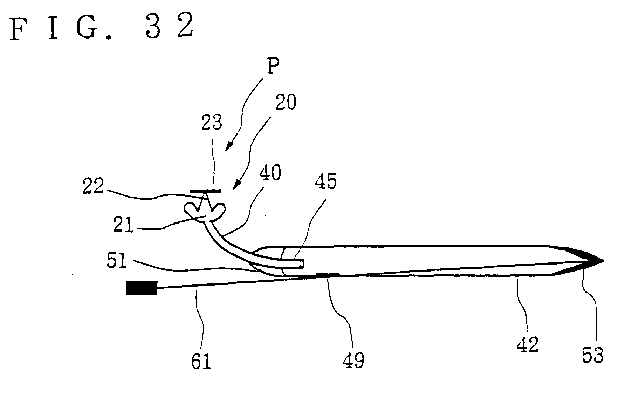

FIG 32 shows one of the way of insertion of the apparatus for intubation in the present invention as to the embodiments shown in FIGS 30∼31.

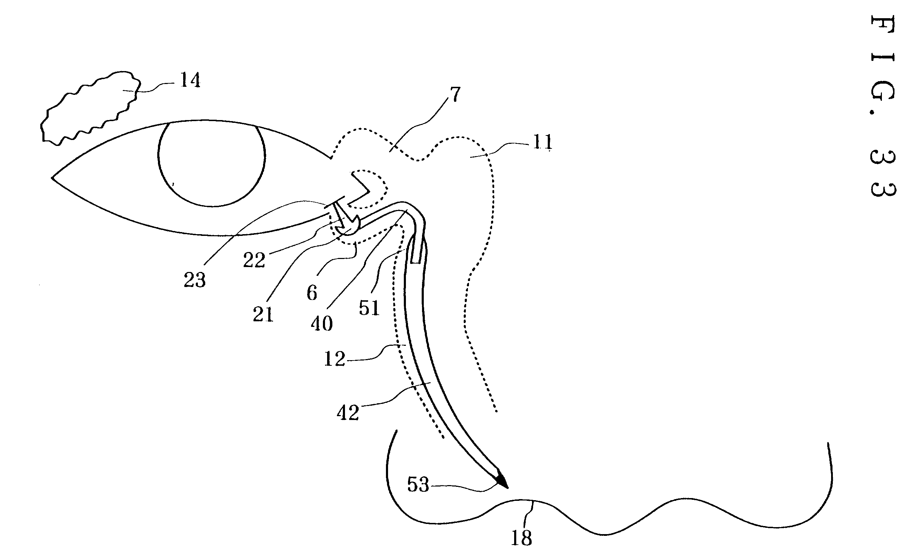

FIG 33 shows one of the post-operative state of placement the apparatus for intubation in the present invention inserted from the lower canaliculus in the embodiments in FIGS 30∼31.

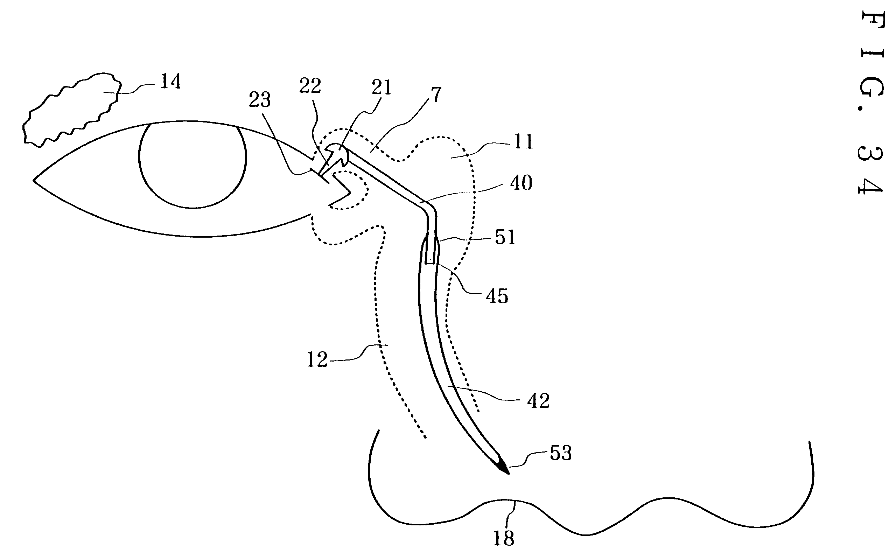

FIG 34 shows one of the post-operative state of placement of the apparatus for intubation in the present invention inserted from the upper canaliculus as to the embodiments in FIGS 30∼31.

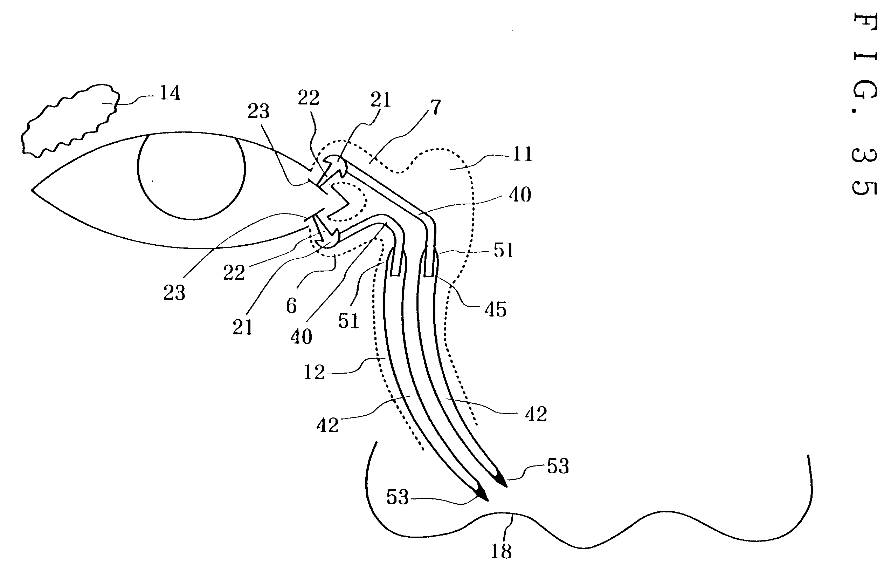

FIG 35 shows one of the post-operative state of placement of the apparatus for intubation in the present invention inserted from the upper and lower canaliculi as to the embodiments in FIGS 30∼31.

In many cases, one of the upper and lower puncta is sufficient to prevent epiphona. Therefore it is superior to place the apparatus for intubation in present intubation in the lower or upper canaliculus after opening of obstructed portion(s) of the lacrimed duct.

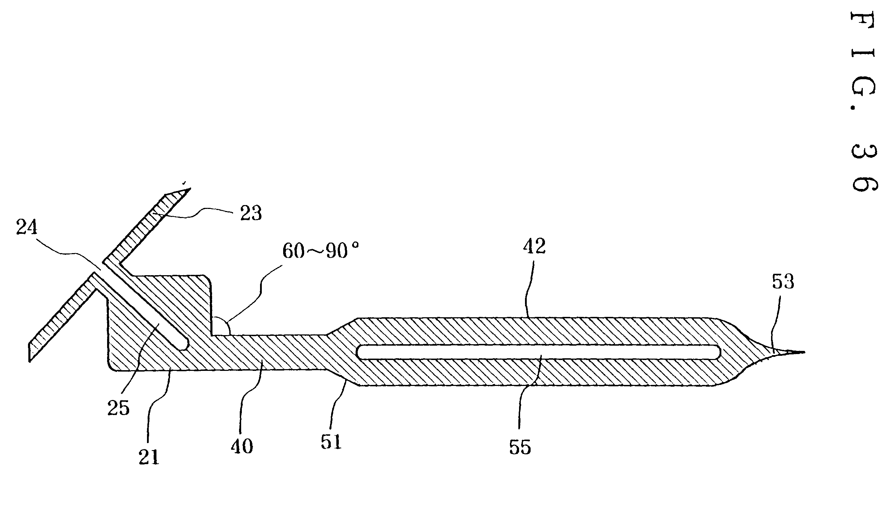

FIGS 36∼37 show still other embodiments where the puntal plug P is attached to an end of the thinner soft rod. The punctal plug P consists of the tip **21** and the brim **23**. The edge **21a** of the tip **21** is round for granulation so as not to easily be induced. The brim **23** is circular, elliptical, in other shapes and made of hard silicone. The posterior end of the thicker hard tube **42** is connected with the other end of the thinner rod **40** so as to form one body. The tip **53** of the thicker tube **42** is sharp pointed and closed. The inner space **25** created in the plug P is connected with the hole of the brim **23**. The inner space **55** is present in the thicker tube 1.0∼1.2mm OD and 0.5mm ID. The junction between the thinner rod **40** and thicker tube **42** is the slope **51** without making any step. And in this embodiments also, the axis of the brim **23** is at a prescribed angle (for example 90∼150 degrees) to the axis of the thicker tube **42**.

In the embodiments in FIGS 36∼37, the tube **42** can be pushed into the lacrimal duct by a probe **61** which inserted into the inner space **55** from the small cut **49** of the thicker tube **42**.

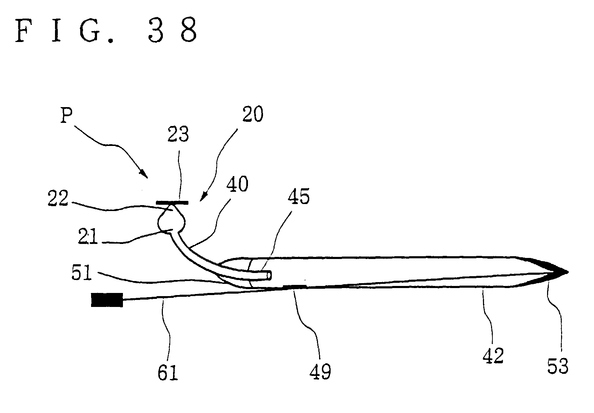

FIG 38 shows one of the way of insertion of the apparatus for intubation in the present invention, as to the embodiments in FIGS 36∼37.

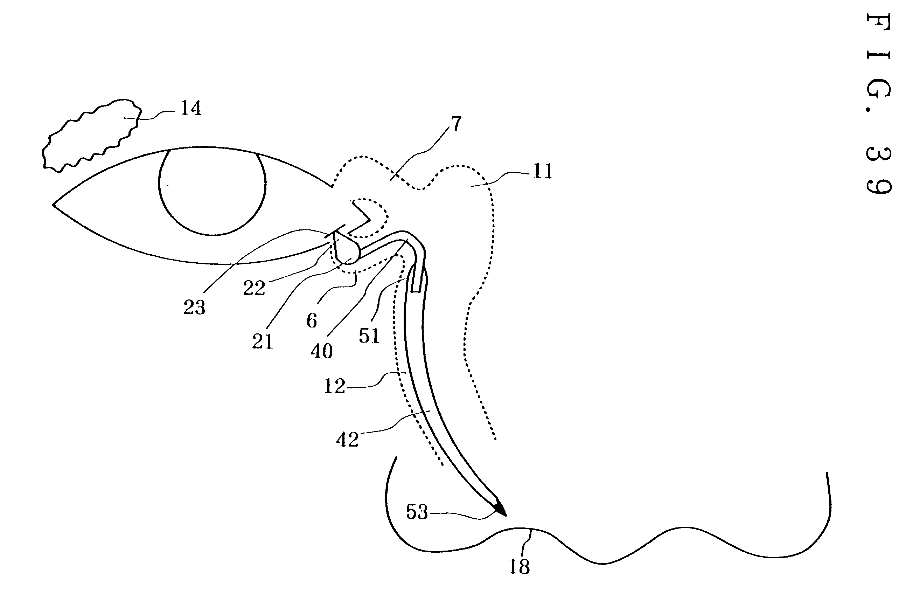

FIG 39 shows one of the post-operative states of placement of the apparatus for intubation in the present invention inserted from the lower canaliculas as to the embodiments in FIGS 36∼37.

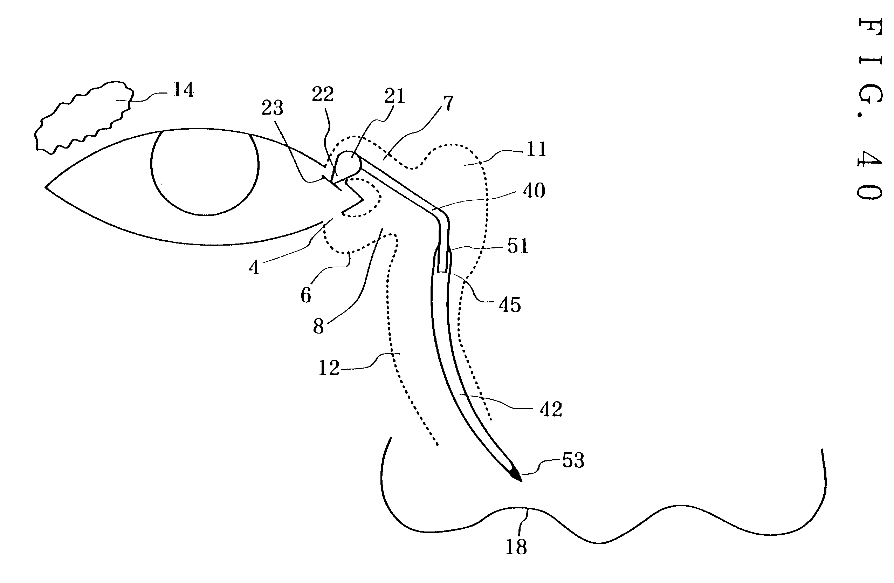

FIG 40 shows one of the post-operative states of placement of the apparatus for intubation in the present invention inserted form the upper canaliculus as to the embodiments in FIGS 36∼37.

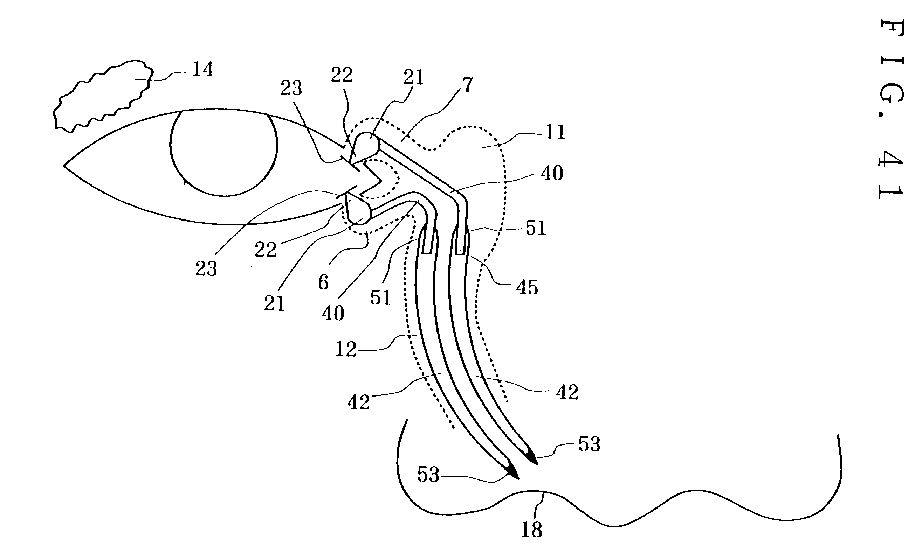

FIG 41 shows one of the post-operative states of placement of the apparatus for intibation in the present invention inserted form the upper and lower cacnaliculi as to the embodiment in FIGS 36∼37.

One of the upper and lower canaliculi is sufficient to prevent epiphora. Therefore, it is superior to place the apparatus for intubation in the present invention in the lower or upper canaliculus, after opening of obstructed portion(s) of the lacrimal duct.

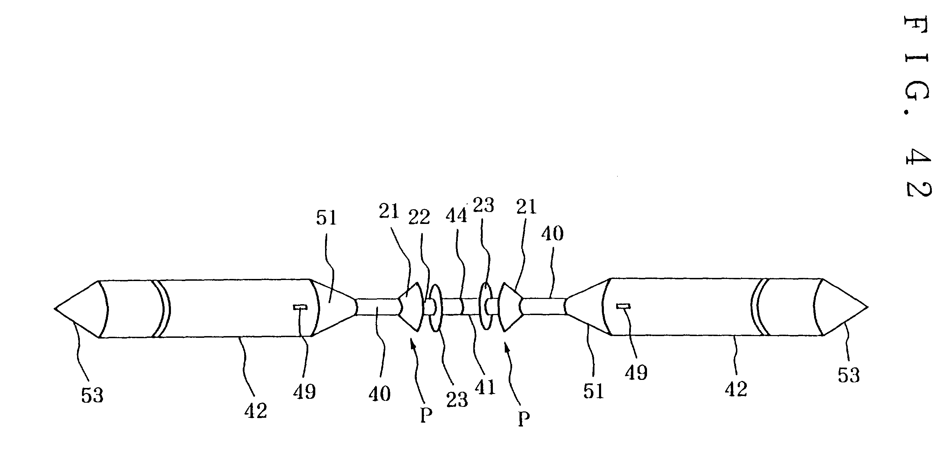

FIGS 42∼43 show still other embodiments in the present invention.

The apparatus for intubation of the lacrimal duct shown in FIGS 42∼43, has a prescribed length of two thinner soft rods **40** and two thicker tubes **42** to be inserted into the lacrimal duct. One end of each thicker tube **42** is closed. Between the two thicker tubes **42**, two thinner rods **40** exist and between the two thinner rods **40**, a thinner rod **41** is present, and the midpoint of the thinner rod **41** has a marking **44**. Junction between the thinner rod **40** and the thicker tube is a slope **51** so as not to make any steps. The tip **53** of the thicker tube **42** is in a conical shape.

The punctal plug P is positioned between two thinner soft rods **40, 41**. The punctal plug P consists of the tip **21**, shaft **22** and brim **23**. The brim is circular, elliptical and in other shapes and made of hard silicone. The plug P has not any inner space. The thicker tube has an inner space **55** and its outer diameter is 1.0∼1.2mm and inner diameter is 0.5mm. The junction between the thinner rod **40** and the thicker tube **42** has the shape **51** without making any steps.

In the embodiment in FIGS 42∼43, the tube **42** can be easily pushed into the lacrimal duct by the probe **61** which is inserted into the inner space from the small cut **49** of the thicker tube **42**.

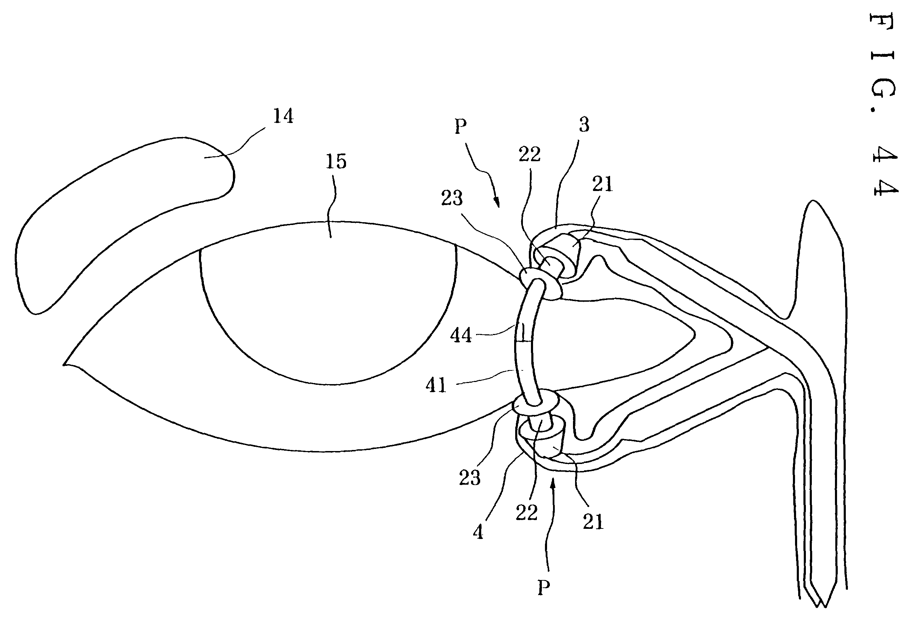

FIG 44 shows one of the ways of insertion from the upper and lower canaliculi, of the apparatus for intubation in the present invention, as to the embodiment sown in FIGS 42∼43.

Finally, the general method of surgery using the apparatus for intubation according to the present invention will be explained.

Before insertion, the obstructed segment(s) of the lacrimal duct is opened by insertion of probe **61**. And in advance, the puncta are dilated by punctal incision at their lateral wall or using a punctal dilator. The tip **53** of the tube **42** enclosing the probe 0.4mm in diameter **61** pushed into the inferior nasal meatus from the lower punctum **2** via the lower canaliculus **4, 6, 8**, common canaliculus **9**, lacrimal sac **11** and nasolacrimal duct **12**. And then, only the apparatus for intubation is left and the probe **61** is removed.

Next, if necessary, another apparatus for intubation is pushed into the lacrimal duct from the upper punctum **1** and in advance, the probe **61** 0.5∼1.mm in diameter is inserted from the upper punctum **1**. The upper punctum is also dilated by punctal incision at the lateral wall or using a punctal dilator. The tip **53** of the tube **42** is pushed into the inferior nasal meatus by the probe **61** which inserted into the thicker tube from the small cut in the same way.

It can be removed easily by holding and pulling the plug P, brim **23** or thinner rod **41** at the upper punctum **1** and the lower punctum **2** using forceps.

Although any of the apparatus for intubation in the present invention is usually used under local anesthesia or general anesthesia using an operating microscope, it can be used simply for many patients under local anesthesia.

Silicone is preferable to make the apparatus for intubation according to the present invention. Silicone is unstimulating and non-toxic to the living body so it is possible to leave in place for a long time.

Unlike the prior arts, the apparatus for intubation according to the present invention, does not require difficult nasal procedure at all, resulting in short operating time and a small burden on patients.

Unlike the prior tubes, it has the stopper and can be bent, to make the tube stable in the lacrimal duct without easy migration. Furthermore, patients feel very little pain.

Although it is easy to insert the apparatus for intubation according to the present invention into the lacrimal duct and easy to remove it, it is not easily dislocated during intubation period.

It is still better to combine the thicker tube with the thinner tube or rod.

The small cuts applied to the thicker tube do not break the tube.

The tube with a sharp pointed tip in conical shape can be easily intubated into the lacrimal duct after punctal dilation with the punctal dilator only without punctal incision.

Using the apparatus for intubation, intubation into the lacrimal duct can be performed more easily. This fact make it possible for doctors to do intubation routinely before resorting to major surgical interventions.

The apparatus for intubation is not easily dislocated compared to the punctal plug when it is used for the treatment of dry eye.

Furthermore the following effects can be gained according to the present invention.
1) It is unnecessary to fix it by suture(s) in monocanalicular intubation.
2) Incidence of granulation arise by the stimulation of the angular portions in the prior punctal plug is decreased by rounding the angular portion.
3) Obstruction between the vertical portion and horizontal portion of canaliculus is not induced. Furthermore, it is useful for a stent of canalicular obstruction and nasolacrimal duct obstruction.
4) By making the brim of the punctal plug elliptical, the brim can be enlarged so as not to stimulate the ocular surface and prevent the plug from migrating into the canalicular.
   By making the tip of the thicker tube sharp pointed, it can be easily inserted from the punctum.
6) Tear fluid cannot enter into the punctum by the brim which adhere to the puncta because the thicker tube is pulled into the lacrimal duct due to the lacrimal drainage function.
7) Punctal occlusion and the treatment of lacrimal duct obstruction and dacryocystitis can be performed simultaneously for patients with dry eye, lacrimal duct obstruction and dacryocystitis.
8) Positioning of the thinner soft tube between the punctal plug and the thicker tube makes the apparatus for intubation stable in the lacrimal duct. Furthermore, making an angle of 90° - 150° between the axises of the punctal plug and the thicker tube makes it stabler.
9) In the apparatus which consists of the thinner and the thicker tubes, attachment of the brim only to the thinner tube also makes it stable.

## Claims

1. An apparatus for intubation of the lacrimal duct (1-12), characterized by:
a thinner tube or rod (40) having a prescribed length, a thicker tube (42) having a prescribed length, extending from one end of said thinner tube or rod (40), and a stopper (P; 23; 22, 23) attached to the other end of said thinner tube or rod (40), wherein the tip (53) of said thicker tube (42) is sharp pointed and closed.

2. An apparatus for intubation of the lacrimal duct (1-12), characterized by:
a thicker tube (42) having a prescribed length, and a stopper (P) attached to the posterior end of said thicker tube (42), wherein the tip (53) of said thicker tube (42) is sharp pointed and closed.

3. An apparatus as defined in claim 1 or 2, characterized in that said stopper (P) is a punctual plug (P).

4. An apparatus as defined in claim 1 or 2, characterized in that said stopper (23) is a brim (23).

5. An apparatus as defined in claim 1 or 2, characterized in that said stopper (23) is a ring (23, 24).

6. An apparatus as defined in any of claims 1 - 5, characterized in that the axis of said stopper (P; 23; 22, 23) is at an angle of preferably 90-150 degrees to the axis of said thicker tube (42).

7. An apparatus as defined in any of claims 1 - 6, characterized in that a small cut (49) runs parallel to the axis of said thicker tube (42) for a probe (62) to be inserted therein.

8. An apparatus as defined in anyone of claims 1 - 6, characterized in that two of said stoppers (P) are connected with said thinner tube or rod (40), each said stopper (P) is attached to the end of said thicker tube (42) or said thinner tube (40).
